# EUROPEAN PATENT APPLICATION

(11) **EP 3 419 239 A1**
(43) Date of publication of application: **26.12.2018**
(21) Application number: 17176783.3
(22) Date of filing: 20.06.2017
(51) Int. Cl.: H04L 29/06, A61B 5/00, H04W 12/06, H04B 10/116, H04B 10/114

(54) **A DEVICE OPERABLE TO PAIR WITH ONE OR MORE OTHER DEVICES AND A METHOD OF OPERATING THE DEVICE**

(71) Applicant: Koninklijke Philips N.V., 5656 AE Eindhoven (NL)
(72) Inventor: GERHARDT, Lutz Christian, 5656 AE Eindhoven (NL); UZUNBAJAKAVA, Natallia Eduardauna, 5656 AE Eindhoven (NL); VAN LIESHOUT, Ron Martinus Laurentius, 5656 AE Eindhoven (NL); JOHNSON, Mark Thomas, 5656 AE Eindhoven (NL); DE BRUIJN, Frederik Jan, 5656 AE Eindhoven (NL); GROB, Timon Rutger, 5656 AE Eindhoven (NL); MEFTAH, Mohammed, 5656 AE Eindhoven (NL); MIGNON, Charles G.M., 5656 AE Eindhoven (NL); VISWESWARA, Ashoka Sathanur, 5656 AE Eindhoven (NL)
(74) Representative: de Haan, Poul Erik

(57) **Abstract**

There is provided a device (100) operable to pair with one or more other devices and a method of operating the device (100). The device (100) comprises a light detector (102) configured to detect a coded light signal from at least one light source that is separate to the one or more other devices. The coded light signal comprises information associated with the one or more other devices. The device (100) also comprises a processor (104) configured to decode the coded light signal that is detected by the light detector (102) to extract the information associated with the one or more other devices and pair the device (100) with at least one of the one or more other devices using the extracted information.

## Description

### TECHNICAL FIELD OF THE INVENTION

The invention relates to the field of devices and, in particular, to a device operable to pair with one or more other devices and a method of operating the device.

### BACKGROUND TO THE INVENTION

Most devices need to be paired with other devices before the devices are able to communicate with each other. An example of devices that need to be paired are wireless devices. Nowadays, many wearable (for example, body-worn) devices, insertable devices, and implantable devices need to establish a communication with a wireless infrastructure for data transfer. For example, these types of devices are often used to communicate data from a patient (or other user of the device) to another device (such as a central server) or vice versa for processing, storage, or other purposes. In order for a communication to be established to transfer data in these cases, the device first needs to be paired with a wireless infrastructure.

In most communications, it is important that data is transferred securely and this is particularly the case when transmitting patient data to other devices (or wireless infrastructures). For example, wireless patient monitoring in hospitals and remote patient monitoring using hospital to home monitoring services, requires a high level of security for the transfer of data between devices. It is particularly important that data is securely transmitted to only those devices that have the correct permission to access the data. There are several techniques that can be used to improve the security of communications. One technique is to use human intervention during the pairing process, while another technique is to program a device with information indicating with which other devices the device is allowed to pair and transfer data. However, both of these techniques result in communication delays and increase the complexity of enabling the communications. This can be particularly undesirable in situations where a frequent and quick pairing process is required (such as in hospitals). It is also difficult to pair devices quickly, effortlessly, and securely where the devices move to multiple locations, such as where the users of the devices move between various physical locations (for example, in and out of different rooms in a hospital, from home to hospital, and so on), since manual intervention is required to pair the moving devices to the correct devices each time the device moves to a new location.

There are various wireless technologies that are available for short-range personal communication purposes, such as Wi-Fi, Bluetooth, and the like. However, even so, both Wi-Fi and Bluetooth enabled devices require first time pairing to another device (such as a hub) using human intervention for data transfer to begin. In these cases, it is also necessary to select on a graphical user interface (GUI) the correct device with which to pair from the many that may be available for pairing. In some cases, a user is required to manually pair a device to another device every time a data transfer operation is initiated.

There are some situations where devices can pair with each other automatically by programming the devices with unique codes (such as a media access control, MAC, address). However, this still requires the devices to be in range of one another. This has a limitation that, each time a new device is placed in or on a body of a subject, the device will need to be programmed with a set of addresses of the devices to which it can connect. This can be prone to errors and addresses may be missed, leading to lost data, in addition to the increased complexity involved, which can be particularly problematic in a healthcare environment. It may be that pairing has to be performed only once when the device pairs to another for the first time. However, in many situations the device does not always pair to the same device. For example, it is often the case that devices used in hospitals and hospital to home monitoring settings need to be changed or replaced regularly and, again, human intervention may then be necessary for the pairing process.

Another technique for pairing is to use radio frequency, RF, waves. However, RF waves disperse in free space and thus lack spatial specificity. Moreover, as RF waves can pass through walls, multiple devices will appear for selection on a GUI. This can increase the risk of errors in the manual intervention that is required to select a device with which to pair and can make the process more time consuming.

In another example, the use of a GUI can in fact be avoided by using near field communication, NFC, to pair devices. However, the devices to be paired using this mechanism need to be brought into close proximity of, or even in contact with, one another (for example, 5 cm apart) for NFC communication to be used to pair the devices for data transfer. In many situations, this is not possible and also poses severe constraints to the ease of pairing devices.

Another method for pairing two devices that suffers from similar drawbacks is disclosed in US 2014/0281547, where one device transmits a light signal to the other device and the light signal corresponds to a private key for use in pairing the devices. Since the light signal transmitted from one of the devices to be paired needs to be detected by the other device to be paired, the detecting device in this situation also needs to be physically close to, and in the line of sight of, the transmitting device in order for pairing to be successful.

There is thus a need for an improved device operable to pair with one or more other devices and an improved method of operating the device to pair with the one or more other devices.

### SUMMARY OF THE INVENTION

As noted above, a limitation with existing approaches for pairing devices is that the approaches are inefficient, complex, insecure, prone to errors, and at risk of failing. It would thus be valuable to have an improved device operable to pair with one or more other devices and an improved method of operating the device to pair with the one or more other devices, which overcomes the existing problems.

Therefore, according to a first aspect of the invention, there is provided a device operable to pair with one or more other devices. The device comprises a light detector configured to detect a coded light signal from at least one light source that is separate to the one or more other devices. The coded light signal comprises information associated with the one or more other devices. The device also comprises a processor configured to decode the coded light signal that is detected by the light detector to extract the information associated with the one or more other devices and pair the device with at least one of the one or more other devices using the extracted information.

In some embodiments, the information associated with the one or more other devices may identify at least one of the one or more other devices with which to pair the device. In some embodiments, the processor may be configured to pair the device with the at least one other device identified by the information. In some embodiments, the coded light signal may further comprise location information for the device and the processor may be configured to determine a location of the device based on the location information.

In some embodiments, the coded light signal may further comprise a code for use in validating the at least one light source from which the coded light signal is detected. In some embodiments, the processor may be configured to confirm with a central server that the code is valid and proceeds to pair the device with the at least one other device only if the validation code is valid.

In some embodiments, the device may comprise a plurality of light detectors configured to detect coded light signals from a plurality of light sources that are separate to the one or more other devices and each light detector may have a different field of detection to detect coded light signals from the plurality of light sources with different strengths. In these embodiments, the processor may be configured to separate the detected coded light signals based on the different strengths of coded light signals detected by the plurality of light detectors.

In some embodiments, the light detector may comprise any one or more of a wavelength filter configured to restrict the light detector to detect coded light signals having a set wavelength, a polarization filter configured to restrict the light detector to detect coded light signals that are polarized in a set direction, and an interference pattern filter configured to restrict the light detector to detect coded light signals having a set interference pattern.

In some embodiments, the processor may be further configured to establish a communication with the at least one other device with which the device is paired.

In some embodiments, the device may be a wearable device or an implantable device. In embodiments where the device is an implantable device, the coded light signal may have a wavelength that penetrates the body to a depth at which the device is located.

According to a second aspect of the invention, there is provided a system comprising at least one device as described above and at least one light source configured to emit coded light signals.

In some embodiments, the at least one light source may comprise any one or more of a light source configured to emit visible coded light signal, a light source configured to emit invisible coded light signals, a stationary light source configured to emit coded light signals, and a moveable light source configured to emit coded light signals.

According to a third aspect of the invention, there is provided a method of operating a device to pair with one or more other devices. The method comprises detecting a coded light signal from at least one light source that is separate to the one or more other devices. The coded light signal comprises information associated with the one or more other devices. The method also comprises decoding the coded light signal that is detected by the light detector to extract the information associated with the one or more other devices and pairing the device with at least one of the one or more other devices using the extracted information.

According to a fourth aspect of the invention, there is provided a computer program product comprising a computer readable medium, the computer readable medium having computer readable code embodied therein, the computer readable code being configured such that, on execution by a suitable computer or processor, the computer or processor is caused to perform the method or the methods described above.

According to the aspects and embodiments described above, the limitations of existing techniques are addressed. In particular, according to the above-described aspects and embodiments, a coded light source that is separate from the devices to be paired is used to pair the devices. Also, as coded light signals have a high spatial specificity due to their properties, the coded light signals do not disperse in free space and can be directed to specific areas. According to the aspects and embodiments described above, it is possible to pair multiple devices quickly, effortlessly, and securely. The devices can thus easily share information for secure pairing such that on-demand pairing can be achieved with the correct devices for secure data transfer.

The pairing of devices according to the above-described aspects and embodiments is also less prone to errors and is more often successful than the existing techniques mentioned earlier. Moreover, pairing by way of the above-described aspects and embodiment is also possible where one or more of the devices to be paired move between locations. Also, the use of a light detector to detect coded light signals to acquire information for use in pairing the device with one or more other devices means that pairing is even at extremely low light levels (for example, due to clothing or skin being present between the light detector of the device and the at least one light source).

There is thus provided an improved device operable to pair with one or more other devices and an improved method of operating the device to pair with the one or more other devices, which overcomes the existing problems.

### BRIEF DESCRIPTION OF THE DRAWINGS

For a better understanding of the invention, and to show more clearly how it may be carried into effect, reference will now be made, by way of example only, to the accompanying drawings, in which:
Figure 1 is a block diagram of a device operable to pair with one or more other devices according to an embodiment;
Figure 2 is an illustration of a system comprising at least one device operable to pair with one or more other devices according to an embodiment;
Figure 3 is a flow chart illustrating a method according to an embodiment;
Figure 4 is a flow chart illustrating a method according to another embodiment;
Figure 5 is an illustration of an in-body device operable to pair with one or more other devices according to an embodiment;
Figure 6 is an illustration of a device comprising a polarization filter according to an embodiment;
Figure 7 is an illustration of a detector arrangement of a device according to an embodiment;
Figure 8 is an illustration of a detector arrangement of a device according to another embodiment; and
Figure 9 is an illustration of a detector arrangement of a device according to another embodiment.

### DETAILED DESCRIPTION OF THE PREFERRED EMBODIMENTS

As noted above, there is provided an improved device operable to pair with one or more other devices and an improved method of operating the device to pair with the one or more other devices, which overcomes existing problems.

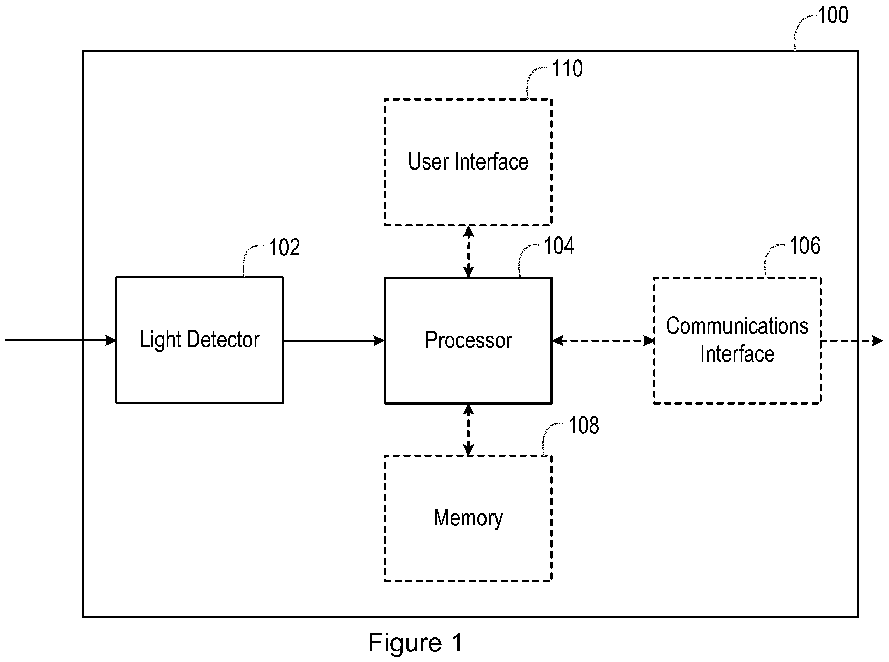

Figure 1 illustrates an example of a device 100 operable to pair with one or more other devices according to an embodiment. In any of the embodiments described herein, the device 100 can be a body-worn device. For example, the device 100 can be an on-body device (such as a wearable device configured to be worn on the body of a user) or an in-body device (such as an implantable device configured to be implanted in the body of a user or an insertable device configured to be inserted into the body of a user, which may be temporary or permanent).

In an embodiment where the device 100 is an on-body device, the device 100 may be in the form of a patch configured to attach to the body of the user. For example, the patch may comprise an adhesive layer configured to adhere to the skin of the user. The patch may, for example, be a disposable patch. In another embodiment where the device 100 is an on-body device, the device 100 may be in the form of a (for example, temporary) tattoo configured to be worn on the body of a user. A tattoo is unobtrusive and may be configured to be ultrathin (such as a film). A tattoo may be configured to conform to the skin contours or topography and may, for example, adhere to the skin by Van der Waals forces. In another embodiment where the device 100 is an on-body device, the device 100 may be in the form of a pendant configured to be worn on the body of a user, such as on a cord (or necklace) to be placed around the neck of the user. Other examples of an on-body device include, but are not limited to, a wrist-worn device 100 (for example, a watch such as a health watch), a belt or strap comprising the device 100, clothing comprising the device 100 (for example, a T-shirt such as a health monitoring T-shirt), or any other form of body-worn device.

In an embodiment where the device 100 is an on-body device, the device 100 may be positioned on or above the surface of skin of the user. In an embodiment where the device 100 is an in-body device, the device 100 may be positioned in the skin surface (for example, in the epidermis or dermis) or below the skin surface (for example, in the stratum corneum). In other embodiments, the device 100 may be a combination of an in-body and an on-body device in that at least part of the device 100 may be positioned in or below the skin surface and at least part of the device 100 may be positioned on or above the skin surface. The device 100 according to any of the embodiments described herein can be an electronic device. For example, the device 100 may be an electronic monitoring device for use in monitoring a user of the device 100. The user of the device 100 can, for example, be a patient, a subject, or any other user.

As illustrated in Figure 1, the device 100 comprises a light detector 102. The light detector 102 is configured to detect a coded light signal from at least one light source that is separate to the one or more other devices. The at least one light source described herein may thus also be referred to as at least one coded light source. The coded light signal that the light detector 102 is configured to detect comprises information associated with the one or more other devices. Examples of a light detector 102 that may be used to detect the coded light signal include, but are not limited to, a photodetector (such as a silicon photodetector), a charge-coupled device (CCD), a photodiode, a camera, scintillator crystals or garnets (which can, for example, convert x-rays into light flashes for pairing), any other type of light detector, or any combination of light detectors, suitable for detecting coded light signals. According to some embodiments, the light detector 102 can be placed at, or on, the surface of the device 100 such that the light detector 102 is exposed to the at least one light source to receive coded light signals from the at least one light source. In some embodiments, the device 100 may comprise a single light detector 102 configured to detect coded light signals. In other embodiments, the device 100 may comprise a plurality of light detectors 102 configured to detect coded light signals.

As illustrated in Figure 1, the device 100 further comprises a processor 104. The processor 104 of the device 100 receives the coded light signal from the light detector 102. In this respect, according to some embodiments, the processor 104 may comprise a coded light receiver (or receiver electronics). The processor 104 also controls the operation of the device 100 and can implement the method described herein. The processor 104 can comprise one or more processors, processing units, multi-core processors or modules that are configured or programmed to control the device 100 in the manner described herein. In particular implementations, the processor 104 can comprise a plurality of software and/or hardware modules, each configured to perform, or are for performing, individual or multiple steps of the method described herein.

Briefly, the processor 104 of the device 100 is configured to decode the coded light signal that is detected by the light detector 102 to extract the information associated with the one or more other devices and to pair the device 100 with at least one of the one or more other devices using the extracted information.

In some embodiments, the one or more other devices to which the device 100 is paired may be the same type of device as the device 100 itself, such as any of those mentioned earlier. In some embodiments, at least one of the other devices may be a different type of device to the device 100 itself. For example, the one or more other devices may comprise any one or more of a server (such as a central server), a hub, a wired or wireless infrastructure, a portable device (such as a laptop, a tablet, a smartphone, or any other portable device), or any other type of device, or any combination of devices. In some embodiments, at least one of the one or more other devices may comprise the same components as the device 100 itself, as described herein with reference to Figure 1. Similarly, in some embodiments, at least one of the one or more other devices may operate in the same manner as the device 100 itself, as described herein.

Optionally, according to some embodiments, as illustrated in Figure 1, the device 100 can also comprise a communications interface (or module or circuitry) 106. The communications interface 106 can be for enabling the device 100 to communicate with (or connect to) the one or more other devices to which the device 100 is paired. Alternatively or in addition, the communications interface 106 can be for enabling the device 100 to communicate with (or connect to) any components, interfaces, units, memories, sensors, or any other devices that are internal or external to the device 100. In any of the embodiments described herein, the communications interface 106 may be configured to communicate with any components, interfaces, units, memories, sensors and devices wirelessly, via a wired connection, or via any other communication (or data transfer) mechanism. In some wireless embodiments, the communications interface 106 may, for example, use radio frequency (RF), Bluetooth, or any other wireless communication technologies, for communications. The device 100 may exchange data (or information) with the one or more other devices to which the device 100 is paired via the communications interface 106.

According to some embodiments, as illustrated in Figure 1, the device 100 can optionally comprise a memory 108. The memory 108 can be configured to store program code that can be executed by the processor 104 to perform the method described herein. Alternatively or in addition to the memory 108 of the device 100, one or more memories 108 may be external to (i.e. separate to or remote from) the device 100. For example, one or more memories 108 may be part of another device. A memory 108 can be configured to store information, data, signals and measurements that are acquired or made by the processor 104 of the device 100 or from any components, units, interfaces, sensors, memories, or devices that are external to the device 100. The processor 104 may be configured to control a memory 108 to store information, data, signals and measurements resulting from the method disclosed herein. For example, the processor 104 may be configured to control a memory 108 to store the information that is extracted from the coded light signal that is detected, an indication of the one or more other devices with which the device 100 is paired using the extracted information, or any other information, or any combination of information resulting from the method described herein.

Optionally, according to some embodiments, as illustrated in Figure 1, the device 100 can comprise a user interface 110. Alternatively or in addition, a user interface 110 may be external to (i.e. separate to or remote from) the device 100. For example, a user interface 110 may be part of another device. A user interface 110 may be configured to receive a user input. In other words, a user interface 110 may allow a user to manually enter data, instructions, or information. The processor 104 of the device 100 may be configured to acquire the user input from one or more user interfaces 110. Alternatively or in addition, a user interface 110 may be for use in providing a user with information resulting from the method described herein. The processor 104 of the device 100 may be configured to control one or more user interfaces 110 to provide information resulting from the method according to the invention. For example, in some embodiments, the processor 104 may be configured to control one or more user interfaces 110 to render (or output, display, or provide) the information that is extracted from the coded light signal that is detected, an indication of the one or more other devices with which the device 100 is paired using the extracted information, or any other information, or any combination of information resulting from the method described herein.

A user interface 110 may be any user interface that enables the rendering (or outputting, displaying, or providing) of information, data or signals to a user of the device 100. Alternatively or in addition, a user interface 110 may be any user interface that enables a user of the device 100 to provide a user input, interact with and/or control the device 100. For example, the user interface 110 may comprise one or more switches, one or more buttons, a keypad, a keyboard, a mouse, a touch screen or an application (for example, on a smart device such as a tablet, a smartphone, or any other smart device), a display or display screen, a graphical user interface (GUI) or any other visual component, one or more speakers, one or more microphones or any other audio component, one or more lights (such as light emitting diode LED lights), a component for providing tactile or haptic feedback (such as a vibration function, or any other tactile feedback component), an augmented reality device (such as augmented reality glasses, or any other augmented reality device), a smart device (such as a smart mirror, a tablet, a smart phone, a smart watch, or any other smart device), or any other user interface, or combination of user interfaces. In some embodiments, the user interface 110 that is controlled to render (or output, display, or provide) information, data or signals of the device 100 may be the same user interface as that which enables the user to provide a user input, interact with and/or control the device 100.

Although not illustrated in Figure 1, according to some embodiments, the device 100 may comprise one or more physiological characteristic (or vital signs) sensors, such as one or more heart rate sensors, one or more respiration rate sensors, one or more skin temperature sensors, or any other physiological characteristic sensors, or any combination of physiological characteristic sensors. Alternatively or in addition, the device 100 may comprise one or more activity sensors, such as one or more accelerometers.

It will be appreciated that Figure 1 only shows the components required to illustrate this aspect of the invention and, in a practical implementation, the device 100 may comprise additional components to those shown. For example, the device 100 may comprise a battery or other power supply for powering the device 100 or means for connecting the device 100 to a mains power supply.

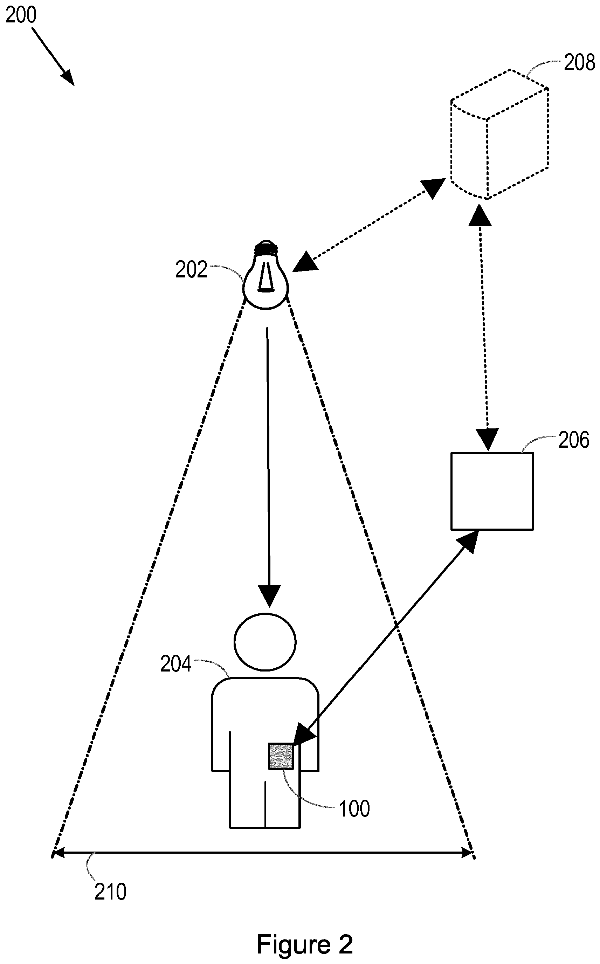

Figure 2 is an illustration of a system 200 comprising at least one device 100 (as described above) according to an embodiment. The device 100 is worn by a user 202. In other words, the device 100 is a body-worn device. Although the user 202 is illustrated as wearing a single device 100, it will be understood that the user 202 may wear more than one device 100 (for example, two or more devices 100), which each may operate in the manner described herein. The device 100 is operable to pair with one or more other devices 204.

As illustrated in Figure 2, the system 200 also comprises at least one light source 206 configured to emit coded light signals. In this illustrated embodiment, the at least one light source 206 comprises a stationary or fixed light source configured to emit coded light signals. However, it will be understood that, in any of the embodiments described herein, the at least one light source 206 can comprise any one or more of a stationary or fixed light source (for example, a permanently installed light source such as a light source attached to or installed in a ceiling or wall of a room) configured to emit coded light signals, a moveable light source (for example, a handheld light source such as a torch or remote control) configured to emit coded light signals, or any other light source configured to emit coded signals, or any combination of light sources configured to emit coded light signals. In a moveable light source embodiment, the moveable light source may be aimed at, and optionally moved over, a user wearing the device 100 (for example, over each side of the user wearing the device 100). This may be performed without illuminating other subjects. A moveable light source embodiment can facilitate the pairing of devices at unconventional locations (or places, or settings). For example, the moveable light source embodiment can facilitate the pairing of devices at locations outside of a healthcare facility (such as outside of a hospital).

Examples of the at least one light source 206 include, but are not limited to, any one or more of a light emitting diode LED (such as an LED operating in the near infrared wavelengths, which may have an emission power greater than 500 mW), or any other type of light source, or any combination of light sources, suitable to emit coded light signals.

The device 100 and, in particular, the light detector 102 of the device 100 is exposed to the coded light signals from the at least one light source 206. More specifically, the at least one light source 206 emits (or sends) coded light signals, which are detected (or received) by the light detector 102 of the device 100. In effect, the at least one light source 206 acts as a location beacon for the devices 100, 204 to be paired, such as a micro-location beacon. The at least one light source 206 provides a location (or micro-location) 208 in which the device 100 can detect a coded light signal from the at least one light source 206 that is separate to the one or more other devices. A micro-location is a physical space of a predetermined radius (for example, a few meters such as 1 meter, 2 meters, 3 meters, 4 meters, or similar) in which the device 100 can detect a coded light signal from the at least one light source 206 that is separate to the one or more other devices 204. The environment (or part of the environment) illuminated by the at least one light source 206 forms the micro-location. The environment may, for example, be a room according to some embodiments. In any of the embodiments described herein, the at least one light source 206 may be for use in pairing multiple devices 100, 204 with each other.

In any of the embodiments described herein, the at least one light source 206 can comprise any one or more of a light source configured to emit visible coded light signals, a light source configured to emit invisible coded light signals (such as infra-red coded light signals), or any other light source configured to emit coded signals, or any combination of light sources configured to emit coded light signals. By using a light source configured to emit invisible coded light signals (such as infra-red coded light signals), the device 100 can be paired with one or more other devices 204 in dark lighting conditions. This can, for example, be the case where a user of the device 100 is asleep in a dark environment. In some embodiments, at least one light source 206 may be configured to combine a coded light signal with functional light. In other words, at least one light source 206 can have two functionalities.

In some embodiments, the system 200 may optionally comprise one or more servers 210. The one or more servers 210 can, for example, comprise at least one central server. The one or more other devices 204 and the server 210 may communicate with each other. Similarly, the at least one light source 206 and the server 210 may communicate with each other.

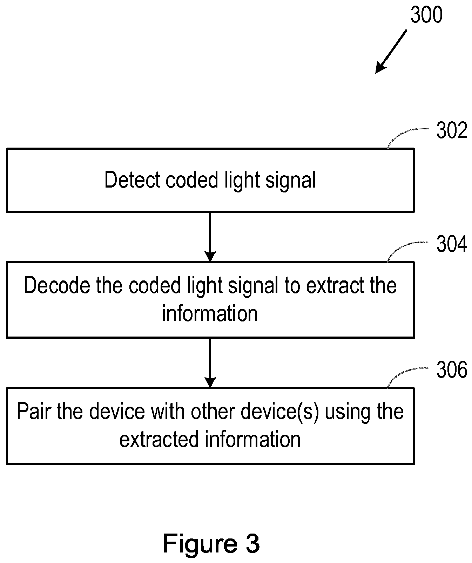

Figure 3 illustrates a method 300 of operating the device 100 described above with reference to Figures 1 and 2, to pair the device 100 with one or more other devices 204, according to an embodiment. The illustrated method 300 of Figure 3 can generally be performed by or under the control of the processor 104 of the device 100. With reference to Figures 2 and 3, at block 302 of Figure 3, a coded light signal is detected from at least one light source 206 that is separate to the one or more other devices 204. Specifically, the light detector 102 of the device 100 detects the coded light signal from the at least one light source 206. The coded light signal comprises information associated with the one or more other devices 204, as described earlier.

At block 304 of Figure 3, the coded light signal that is detected by the light detector 102 is decoded to extract the information associated with the one or more other devices 204. In any of the embodiments described herein, the extracted information can comprise any information relating to the one or more other devices 204 that can be used in pairing the device 100 with the one or more other devices 204. In some embodiments, for example, the information associated with the one or more other devices 204 identifies at least one of the one or more other devices 204 with which to pair the device 100. In these embodiments, the processor 104 can be configured to pair the device 100 with the at least one other device 204 identified by the information.

In some embodiments, the extracted information can comprise secure information. For example, the extracted information may comprise a unique identifier for the one or more other devices 204. Examples of a unique identifier include, but are not limited to, a code that is unique to a device, such as a media access control (MAC) address assigned to a device, a coded light data pattern, or any other unique identifier, or any combination of unique identifiers. In this way, the extracted information can securely indicate one or more other devices 204 to which the device 100 is to pair.

In some embodiments, the extracted information can also comprise other information such as information associated with (or required for) pairing the device 100 with one or more other devices 204, setting up a connection (such as a secure connection) for the device 100 to the one or more other devices 204 with which the device 100 is paired, and/or the transmission of data (or information) between the device 100 and the one or more other devices 204 with which the device 100 is paired. In any of the embodiments herein, the device 100 may be paired to one or more other devices 204 in order for the device 100 to communicate with the one or more other devices 204 and/or to allow the transfer or exchange of data (or information) between the device 100 and the one or more other devices 204.

At block 306 of Figure 3, the device 100 is paired with at least one of the one or more other devices 204 using the extracted information. In some embodiments, the device 100 may remain paired with at least one of the one or more other devices 204 only while the device 100 is in the vicinity or a predefined range of the at least one light source 206. In these embodiments, the device 100 may be unpaired when the device 100 leaves the vicinity or is outside the predefined range of the at least one light source 206. For example, in embodiments where the at least one light source 206 is fixed light source in a room (which may also be referred to as a coded light room), the device 100 may remain paired with at least one of the one or more other devices 204 only while the device 100 is inside the room and may be unpaired when the device 100 (or the user wearing the device 100) leaves the room. In some embodiments, where the light detector 102 of the device 100 does not detect a coded light signal for a predefined time period (for example, due to the user that is wearing the device 100 being outside a room comprising the at least one light source 206), the device 100 may be unpaired.

In some embodiments, the coded light signal can be used to enable repeated pairing of the device 100 with the one or more other devices 204. This can ensure that the pairing of the device 100 with the one or more other devices 204 is stopped when the device 100 is removed from the interaction with the at least one light source 206 (for example, when the user that is wearing the device 100 moves from a room comprising the at least one light source 206 to another room). In any of the embodiments disclosed herein where the device 100 is unpaired, the device 100 may be unpaired by removing or erasing the extracted information (for example, the unique code such as the MAC address) from a memory 108 of the device 100.

Although not illustrated in Figure 3, in any of the embodiments described herein, the method may further comprise establishing a communication with the at least one other device 204 with which the device 100 is paired. More specifically, the processor 104 of the device can be further configured to establish the communication with the at least one other device 204 with which the device 100 is paired. The method may also further comprise transferring or transmitting data (or information) between the device 100 and the at least one other device 204 with which the device 100 is paired. For example, the device 100 may transfer or transmit data (or information) to the at least one other device 204 with which the device 100 is paired, the at least one other device 204 with which the device 100 is paired may transfer or transmit data (or information) to the device 100, or both.

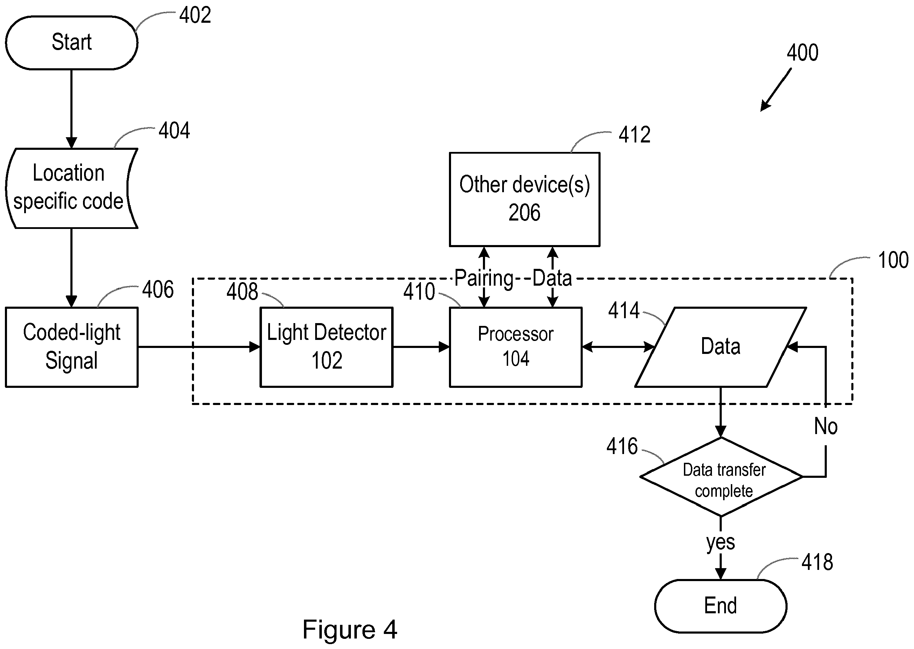

Figure 4 illustrates a method 400 of operating the system 200 described above with reference to Figure 2 that comprises the device 100, to pair the device 100 with one or more other devices 204, according to an example embodiment. The illustrated method 400 of Figure 4 can generally be performed by or under the control of a processor, where at least some of the method can be performed by or under the control of the processor 104 of the device 100. With reference to Figures 2 and 4, at block 402 of Figure 4, the process is started or initiated. At block 404 of Figure 4, the at least one light source 206 codes a light signal with information associated with the one or more other devices 204. In this illustrated example embodiment, the at least one light source 206 codes the light signal with a location specific code. Then, at block 406 of Figure 4, the at least one light source 206 transmits a coded light signal.

The device 100 is exposed to the coded light signal from the at least one light source 206 and, at block 408 of Figure 4, the light detector 102 of the device 100 detects the coded light signal. In other words, the method at block 302 of Figure 3 is performed, which will not be repeated here but will be understood to apply. At block 410 of Figure 4, the processor 104 of the device 100 decodes the coded light signal that is detected by the light detector 102 to extract the information associated with the one or more other devices 204. In other words, the method at block 304 of Figure 3 is performed, which will not be repeated here but will be understood to apply. At block 412 of Figure 4, the processor 104 of the device 100 pairs the device 100 with at least one of the one or more other devices 204 using the extracted information. In other words, the method at block 306 of Figure 3 is performed, which will not be repeated here but will be understood to apply.

At block 414 of Figure 4, data is transferred between the device 100 and the one or more other devices 204 with which the device 100 is paired. At block 416 of Figure 4, it is checked whether data transfer between the device 100 and the one or more other devices 204 is complete. If not, then the data transfer between the device 100 and the one or more other devices 204 with which the device 100 is paired continues at block 414 of Figure 4. When the data transfer between the device 100 and the one or more other devices 204 is complete, the process ends at block 418 of Figure 4.

In any of the embodiments described herein, in addition to the information described earlier that is extracted from the coded light signal, the coded light signal may further comprise a code for use in validating the at least one light source 206 from which the coded light signal is detected. Thus, in effect, the at least one light source 206 can emit (or transmit) a validation code. This validation code may, for example, be a rolling code. For example, the validation code may be a one-time password or value (such as an encrypted code) that expires or changes after a predefined time period or at set time intervals. A validation code can provide secure localization of the at least one light source 206. The coded light signals from the at least one light source 206 may be read and replicated by another light source. For example, another light source can act as a duplicated light source, pretending to be the authentic light source 206, to mislead the device 100 and this can result in the privacy of data (or information) transmitted by the device 100 being compromised. This risk of a breach in the privacy and security of data (or information) can be avoided through use of the validation code. In particular, the validation code can make it difficult for sources external to the system 200 to replicate the at least one light source 206. For example, it can be particularly difficult for an external source to replicate a validation code that changes at set time intervals.

In some embodiments, one or more of the servers 210 (or one or more central servers 210) of the system 100 may run an algorithm to generate the validation code. For example, one or more of the servers 210 can be a time keeping server that runs an algorithm to generate a one-time password (or value) that expires or changes after a predefined time period or at set time intervals. The one or more servers 210 may first be synchronized in time with the at least one light source 206. In some embodiments, this can be achieved in a commissioning phase or with a continuous connection between the at least one light source 206 and the one or more servers 210 (for example, using power over ethernet (PoE) technology). Once at least one light source 206 and the one or more servers 210 are time synchronized, the at least one light source 206 and the one or more servers 210 share a common sequence code (or number) that can then be used to generate the validation code. The validation code can be generated with any one or more of location information for the at least one light source 206, the common sequence code between the at least one coded light source 206 and the one or more servers 210, and a current time. Where the validation code is generated with location information for the at least one light source 206, the validation code may be referred to as a location code.

The at least one light source 206 emits (or transmits) the validation code in the coded light signal, which is detected by the light detector 102 of the device 100. The light detector 102 of the device 100 thus receives the validation code from the at least one light source 206. The processor 104 of the device 100 may then use the validation code to establish communications with the one or more other devices 204 with which the device 100 is to be paired according to the method described herein. For example, in some embodiments, the processor 104 of the device 100 may be configured to confirm or check with one or more of the servers 210 (or, more specifically, the server 210 used in generating the validation code) that the validation code is in fact valid.

In these embodiments, the processor 104 of the device 100 may proceed to pair the device with the at least one other device 204 only if the validation code is valid. In this way, the device 100 will only exchange data (or information) with the one or more other devices 204 or the one or more servers 210 if the validation code is valid. The use of the validation code makes it difficult, if not impossible, for any external source to replicate, or pretend to be, the at least one light source 206. This ensures that the communication links between the device 100, the at least one light source 206, the one or more other devices 204, and the one or more servers 210, are secure. The above-described method can thus be used to generate a secure validation (for example, location) code for the at least one light source 206.

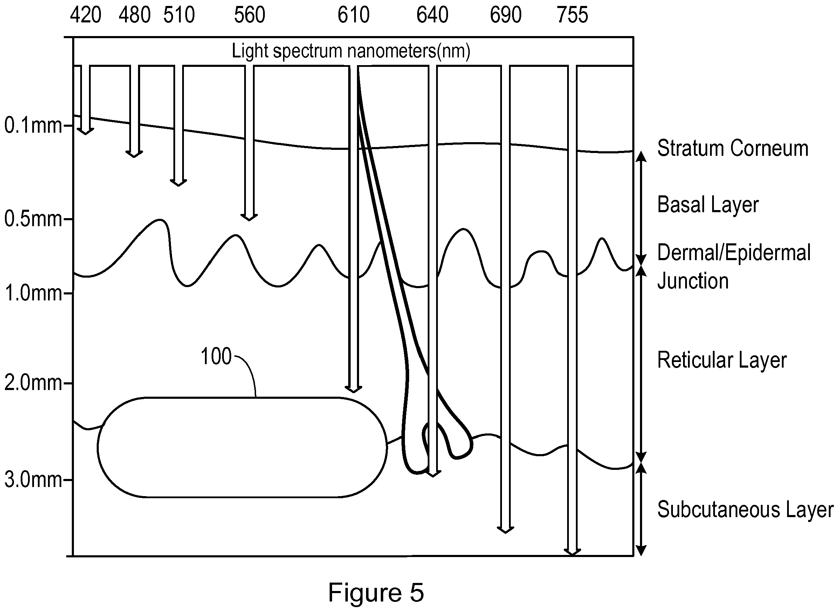

Figure 5 is an illustration of an in-body device 100 operable to pair with one or more other devices 204 according to an embodiment. In this embodiment, the in-body device 100 can be an implantable or insertable device. As illustrated in Figure 5, in embodiments in which the device is an in-body device 100, the coded light signal from the at least one light source 206 can have a wavelength that penetrates the body (or more specifically, the skin, such as the top layers of skin) of the user 202 to a depth at which the device 100 is located. The device 100 may be located (for example, partially) below the upper skin layers, such as the stratum the corneum, the epidermis, and the upper dermis. All of these layers of skin will attenuate the coded light signal. Thus, in order to allow the coded light signal from the at least one light source 206 to reach the device 100 to be detected by the light detector 102 of the device 100, a dedicated range of light parameters (such as a dedicated wavelength, intensity, operation mode (e.g. pulsed, continuous illumination) and/or illumination patterns may be used.

In the illustrated example of Figure 5, the coded light signal is transmitted from the at least one light source 206 in a certain wavelength to be received by the device 100 in the body of the user 202. In this way, the light detector 102 of the device 100 can detect the coded light signal from the at least one light source 206 within the body in which the device 100 is implanted or inserted. In some embodiments, the at least one light source 206 may be located directly under the epidermal-dermal junction (which may be, for example, approximately 0.3 to 0.5 mm under the skin surface) or in the dermis (which may be, for example, approximately 0.5 to 2.0 mm under skin surface) where blood micro-capillaries can be accessed, interstitial fluid, and larger blood vessels for implantable biomarker measurements. In an example, a blue to near-infrared wavelength (such as a wavelength between 400nm and 2000nm) may be used for a coded light signal to allow the coded light signal to penetrate the upper skin layers, as shorter wavelength range is typically dominated by melanin and blood absorption. In another example, a red to near-infrared wavelength (such as a wavelength between 600nm and 1800nm) may be used for the coded light signal to avoid strong absorption by melanin, blood, and water. In some embodiments, a wavelength between 400 nm and 600 nm may be used for the coded light signal. According to some embodiments, the wavelength that is selected can depend on the skin layer thicknesses of the user of the device 100 (which can vary depending on the body region), the age of the user of the device 100, and/or the effect of clothing worn by the user of the device 100 It is also possible, due to the difference in penetration depth of different wavelengths into the skin, to distinguish between the pairing of on-body devices (for example, surface devices) 100 and the pairing of in-body device (for example, subcutaneous devices) 100.

In some embodiments, the wavelength of the coded light signal from the at least one light source 206 may be selected based on the clothing worn by the user 202 of the device 100 under which the device 100 is located. For example a near-infrared wavelength (such as 700nm or above) may be selected to allow the coded light signal from the at least one light source 206 to penetrate clothes. More specifically, a wavelength in a range of between 700nm and 1400nm, or in a narrower range of between 700nm and 1000nm, may be selected since the absorption of light by both polyester and cotton are low. In another example, a wavelength of 1300nm (or approximately 1300nm) may be selected since the absorption of light by both by tissue or skin and clothes is low. As most wavelengths are not completely blocked by clothing, it is possible for the light detector 102 of the device 100 to detect coded light signals from the at least one light source 206 through clothing.

In any of the embodiments described herein, the coded light signal may further comprise a code associated with the user 202 of the device 100, such as a code that is acquired from an electronic health record (EHR) of the user 202 of the device 100. This code can, for example, be in the form of a pre-programed code embedded in the device 100, or may be enabled through use of a filter (such as a wavelength filter, a polarization filter, a light interference pattern filter, or any other filter, or combination of filters) placed over, or on top of, the light detector 102 of the device 100.

In an embodiment that uses a wavelength filter, the light detector 102 of the device 100 can comprise the wavelength filter and the wavelength filter can be configured to restrict the light detector 102 to detect coded light signals having a set wavelength. For example, a wavelength filter may be a light reflector configured to reflect light of a specific wavelength or a light absorber configured to absorb light of a specific wavelength. The wavelength filter may, for example, be integrated in the device 100. By using a wavelength filter, only coded light signals having one or more specific wavelengths are allowed to enter or reach the light detector 102 of the device 100. A wavelength filter may be in the form of an optical filter. For example, a wavelength filter can comprise any one or more of an absorptive filter, a dichroic filter, a monochromatic filter, or any other wavelength filter, or any combination of wavelength filters. In some embodiments, different wavelength filters may be used in different devices 100 to minimize or avoid crosstalk as the devices 100 will respond only to coded light signals that have a specific wavelength. In this way, it is possible to provide unique pairing for different devices 100. Alternatively or in addition to a wavelength filter, the light detector 102 of the device 100 may comprise one or more other filters.

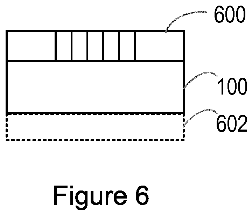

Figure 6 is an illustration of a device 100 comprising a polarization filter 600 according to an embodiment. In this embodiment, the light detector 102 (not shown) of the device 100 can comprise the polarization filter 600. In some embodiments, the polarization filter 600 maybe an in-built polarization filter. The polarization filter can be configured to restrict the light detector 102 of the device 100 to detect coded light signals that are polarized in a set direction. In this illustrated example embodiment, the device 100 is an on-body device 100 that comprises an adhesive layer 602 for adhering to the skin of the user 202. However, as described herein, it will be understood that other types of device 100 and other configurations for the device 100 are also possible.

In embodiments where the light detector 102 of the device 100 comprises a polarization filter 600, the pairing of the device 100 is performed using polarized light. More specifically, a device 100 comprising a polarization filter 600 will respond only to a specific light polarization direction. In some embodiments, different polarization filters 600 may be used in different devices 100 to minimize or avoid crosstalk as the devices 100 will respond only to (coded) light signals that have a specific light polarization direction. In this way, it is possible to provide unique pairing for different devices 100.

In some embodiments, the light detector 102 (or the processor 104) of the device 100 can comprise an interference pattern filter, which may also be referred to as a light interference pattern filter. In embodiments where the light detector 102 (or processor 104) of the device 100 comprises an interference pattern filter, the pairing of the device 100 is performed using unique light interference patterns. More specifically, an interference pattern filter can be configured to restrict the light detector 102 (or the processor 104) of the device 100 to detect coded light signals having a set (or unique) interference pattern. In some embodiments, a set (or unique) light pattern may be recognized by the light detector 102 or an external charge coupled device (CCD) camera. In this way, a device 100 comprising an interference pattern filter responds only to a specific light interference pattern. For example, the light detector 102 (or the processor 104) of the device 100 comprising the interference pattern filter will recognize only a unique light interference pattern. In some embodiments, different interference pattern filters may be used in different devices 100 to minimize or avoid crosstalk as the devices 100 will then respond only to coded light signals that have a specific light interference pattern. In this way, it is possible to provide unique pairing for different devices 100.

In a related embodiment where the device 100 is in the form of an (for example, ultra-thin) electronic skin tattoo, the tattoo may comprise a plurality of (for example, thin) electrically conductive wires. In some embodiments, the plurality of electrically conductive wires may be integrated in a (for example, soft) polymer matrix. In these embodiments, the wires themselves may be used as a diffraction grating or a wire grid polarizer.

In any of the embodiments described herein, the device 100 can comprise a plurality of light detectors 102 configured to detect coded light signals from a plurality of light sources 206 (which may, for example, produce unique light interference patterns) that are separate to the one or more other devices 204. In these embodiments, each light detector 102 may have a different field (for example, a different angular field) of detection or reception to detect coded light signals from the plurality of light sources 206 with different strengths. For example, point-wise light detectors 102 may be mounted on the device 100. This spatial configuration of light detectors 102 causes each light detector to detect coded light signals from multiple light sources 206 with a different strength. By composing a separate signal from each of the light detectors 102, different linear combinations of the multiple coded light signals detected allows isolation of each of the multiple coded light signals. The processor 104 of the device 100, according to these embodiments, may be configured to separate the detected coded light signals based on the different strengths of coded light signals detected by the plurality of light detectors 102. In some embodiments, the strongest coded light signal intensity detected by the plurality of light detectors 102 may be used in the method described herein (namely, the information used in pairing the device 100 may be extracted from the strongest coded light signal), while the other coded light signals detected may be treated as distortions.

In any of the embodiments described herein, the coded light signal may further comprise location information for the device 100. In these embodiments, the processor 104 may also be configured to determine a location of the device 100 based on the location information. In some embodiments, location information may comprise a location specific coded light data pattern. According to some embodiments, the location information may be used by the processor 104 of the device 100 to securely establish communications with the one or more other devices 204. As the location of the device 100 can be determined, it is possible to track the device 100 and/or provide navigation guidance to the device 100. Thus, the system 200 disclosed herein can aid in the localization of a device 100, where the coded light signals provide localization information for the device 100.

As previously mentioned, in some embodiments, the device 100 may be a body worn device operable to pair with one or more other devices 204. In these embodiments, the at least one light source 206 may be configured to project structured or coded light patterns on the user 202 and thus also on the surface of the on-skin or in-skin device 100 worn on the body of the user 202. In this way, the device 100 may be recognized by the at least one light source 206 and this information on the device 100 may be used in pairing the device 100 with one or more other devices 204. For example, the light source 206 may code the light signal that it emits to the light detector 102 of the device 100 with information based on the identity of the device 100 that it determines from the projected light patterns.

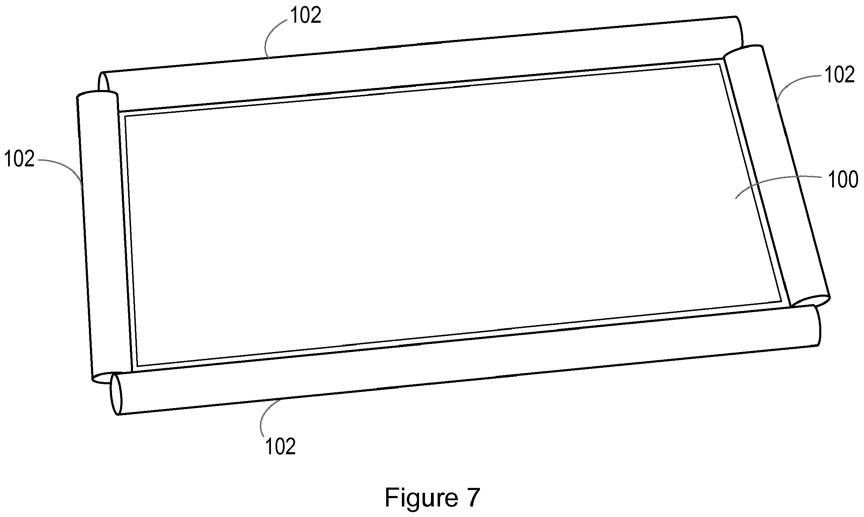

As described earlier, the device 100 may be an on-body device, through-body or an in-body device and can take any suitable form for these applications. Examples of the form that an on-body device 100 may take are provided in Figures 7, 8 and 9 to illustrate possible arrangements for one or more light detectors 102 of the device 100.

Figure 7 is an illustration of a detector arrangement of a device 100 according to an embodiment. As illustrated in the example embodiment of Figure 7, one or more light detectors 102 may be located around the perimeter or outer edge of the device 100 to detect the coded light signal from the at least one light source 206. According to this example embodiment, the one or more light detectors 102 may each be directed to a different incident angle. The one or more light detectors 102 may be cylindrically shaped. The device 100 may, for example, be configured to be attached (for example, clipped onto) clothing worn by the user 202.

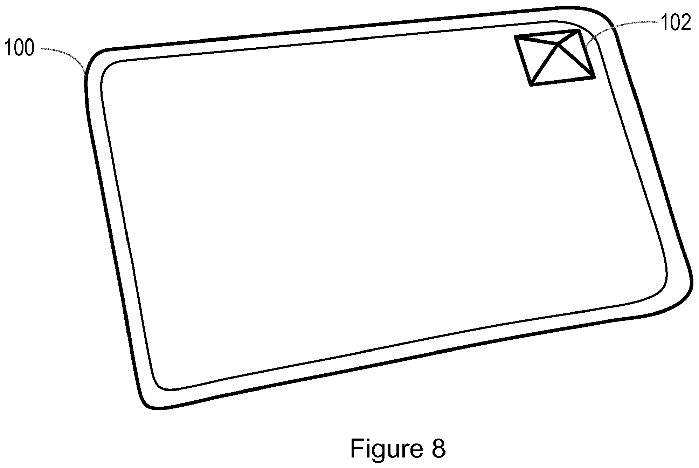

Figure 8 is an illustration of a detector arrangement of a device 100 according to another embodiment. As illustrated in the example embodiment of Figure 8, one or more light detectors 102 may be located on a surface of the device 100 to detect the coded light signal from the at least one light source 206. According to this example embodiment, the one or more light detectors 102 may be arranged in on a faceted element, such as in a pyramid shape, or any other faceted element. In some embodiments, the light detectors 102 may each be located on a different face of the faceted element such that each light detector 102 detects a coded light signal at a different incident angle. The device 100 may, for example, be configured to be attached (for example, clipped onto) clothing worn by the user 202.

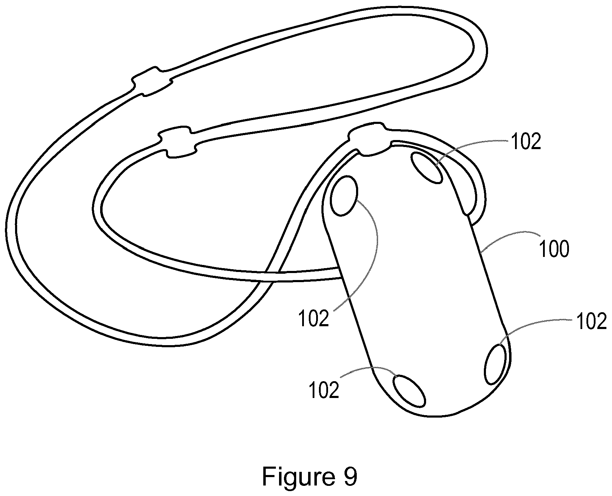

Figure 9 is an illustration of a detector arrangement of a device 100 according to another embodiment. As illustrated in the example embodiment of Figure 9, the device 100 may be in the form of a pendant on a cord to be worn around the neck of the user 202 and the one or more light detectors 102 may be suitably located on the pendant to detect the coded light signal from the at least one light source 206. Each of the light detectors may be oriented at a different incident angle. This form of device 100 may be primarily targeted at fall-detection according to some embodiments. For example, the device 100 may further comprise a button operable to generate an alarm.

Although not illustrated, in another example embodiment, the one or more light detectors 102 may be comprised in (for example, provided on or embedded in) a head-mounted device 100, with each of the light detectors 102 at a different positon on the head-mounted device 100. The detection of the different coded light signals detected around the head of the user 202 may, in some embodiments, benefit from motion sensing and tracking functions embedded in the head-mounted device.

Although examples for the form of an on-body device 100 and arrangements for one or more light detectors 102 of the device 100 have been provided with reference to Figures 7, 8 and 9, it will be understood that the device 100 may instead take any other form, that in-body devices are also possible, and that the one or more light detectors 102 of the device 100 may instead be arranged in any other way suitable to detect coded light signals from at least one light source 206.

There is therefore provided herein an improved device 100 operable to pair with one or more other devices 204 and an improved method of operating the device 100 to pair with the one or more other devices 204. The device 100 and method described herein can be useful in any situations in which devices need to be paired. For example, the device 100 and method described herein can be particularly useful for implants and insertable devices, wearable devices, devices comprising physiological characteristic sensors (for example, vital signs sensors such as heart rate sensors, respiration rate sensors, skin temperature sensors, or similar), devices comprising activity sensors, or any other devices, or any combinations of those devices.

The device 100 and method described herein can be useful in a medical environment (for example, in a healthcare facility such as a hospital, or similar), or at home. The data (or information) exchanged following the pairing of devices in the manner described herein can be useful in a variety of situations. For example, the data may comprise clinical data that is transmitted by the device 100 to the one or more other devices 204 with which the device 100 is paired for processing (such as on a cloud-based platform) and can support a healthcare professional in clinical decisions. However, it will be understood that the device 100 and method described herein is also suitable in any other situation where the pairing of devices for data exchange is useful.

There is also provided a computer program product comprising a computer readable medium, the computer readable medium having computer readable code embodied therein, the computer readable code being configured such that, on execution by a suitable computer or processor, the computer or processor is caused to perform the method or methods described herein.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A device (100) operable to pair with one or more other devices (204), the device (100) comprising:
a light detector (102) configured to detect a coded light signal from at least one light source (206) that is separate to the one or more other devices (204), wherein the coded light signal comprises information associated with the one or more other devices (204); and
a processor (104) configured to decode the coded light signal that is detected by the light detector (102) to extract the information associated with the one or more other devices (204) and pair the device (100) with at least one of the one or more other devices (204) using the extracted information.

2. A device (100) as claimed in claim 1, wherein the information associated with the one or more other devices (204) identifies at least one of the one or more other devices (204) with which to pair the device (100).

3. A device (100) as claimed in claim 2, wherein the processor (104) is configured to pair the device (100) with the at least one other device (204) identified by the information.

4. A device (100) as claimed in any one of the preceding claims, wherein the coded light signal further comprises location information for the device (100) and the processor (104) is configured to determine a location of the device based on the location information.

5. A device (100) as claimed in any one of the preceding claims, wherein the coded light signal further comprises a code for use in validating the at least one light source (206) from which the coded light signal is detected.

6. A device (100) as claimed in claim 5, wherein the processor (104) is configured to confirm with a central server (210) that the code is valid and proceeds to pair the device with the at least one other device (204) only if the validation code is valid.

7. A device (100) as claimed in any one of the preceding claims, wherein the device (100) comprises:
a plurality of light detectors (102) configured to detect coded light signals from a plurality of light sources (206) that are separate to the one or more other devices (204), each light detector (203) having a different field of detection to detect coded light signals from the plurality of light sources with different strengths; and
wherein the processor (104) is configured to separate the detected coded light signals based on the different strengths of coded light signals detected by the plurality of light detectors (102).

8. A device (100) as claimed in any one of the preceding claims, wherein the light detector (102) comprises any one or more of:
a wavelength filter configured to restrict the light detector (102) to detect coded light signals having a set wavelength;
a polarization filter configured to restrict the light detector (102) to detect coded light signals that are polarized in a set direction; and
an interference pattern filter configured to restrict the light detector (102) to detect coded light signals having a set interference pattern.

9. A device (100) as claimed in any one of the preceding claims, wherein the processor (104) is further configured to establish a communication with the at least one other device (204) with which the device (100) is paired.

10. A device (100) as claimed in any one of the preceding claims, wherein the device (100) is a wearable device or an implantable device.

11. A device (100) as claimed in claim 10, wherein the device (100) is an implantable device and the coded light signal has a wavelength that penetrates the body to a depth at which the device (100) is located.

12. A system (200) comprising:
at least one device (100) as claimed in any one of the preceding claims; and
at least one light source (206) configured to emit coded light signals.

13. A system (200) as claimed in claim 12, wherein the at least one light source (206) comprises any one or more of:
a light source configured to emit visible coded light signals;
a light source configured to emit invisible coded light signals;
a stationary light source configured to emit coded light signals; and
a moveable light source configured to emit coded light signals.

14. A method (300) of operating a device (100) to pair with one or more other devices (204), the method comprising:
detecting (302) a coded light signal from at least one light source (206) that is separate to the one or more other devices (204), wherein the coded light signal comprises information associated with the one or more other devices (204);
decoding (304) the coded light signal that is detected by the light detector (102) to extract the information associated with the one or more other devices (204); and
pairing (306) the device with at least one of the one or more other devices (204) using the extracted information.

15. A computer program product comprising a computer readable medium, the computer readable medium having computer readable code embodied therein, the computer readable code being configured such that, on execution by a suitable computer or processor, the computer or processor is caused to perform the method of any one of claims 1 to 14.
